# EUROPEAN PATENT APPLICATION

(11) **EP 1 013 261 A1**
(43) Date of publication of application: **28.06.2000**
(21) Application number: 99310238.3
(22) Date of filing: 16.12.1999
(51) Int. Cl.: A61K 7/26, A61K 7/16

(54) **Composition of mouth spray**

(30) Priority: 18.12.1998 JP 36083398
(71) Applicant: MITSUI & CO., LTD., Chiyoda-ku Tokyo-to 100-0004 (JP)
(72) Inventor: Maki, Hisako, Mitsui & Co. Ltd., Tokyo 100-0004 (JP); Mori, Masao, Mitsui Norin Co. Ltd., Fijieda-shi, Shizuoka 426-0133 (JP); Fukai, Katsuhiko, Mitsui Norin Co. Ltd., Fijieda-shi, Shizuoka 426-0133 (JP); Hara, Yukihiko, Mitsui Norin Co. Ltd., Fijieda-shi, Shizuoka 426-0133 (JP)
(74) Representative: Samuels, Lucy Alice

(57) **Abstract**

A composition of mouth spray is provided, which comprises tea-polyphenol, 1-menthol and/or 1-carvone. A concentration of the tea-polyphenol in the composition of mouth spray is preferably from 10 to 20000 ppm. The tea-polyphenol is preferably at least one member selected from the group containing (+) -catechin, (-) -catechin, (+) -gallocatechin, (+) -epigallocatechin, (+) -gallocatechin gallate, (+) -epigallocatechin gallate, (-) -epicatechin, (-)-epicatechin gallate, (-) -catechin gallate, (-) -epigallocatechin, (-) -gallocatechin, (-) -epigallocatechin gallate, (-)-gallocatechin gallate, theaflavin monogallate A, theaflavin monogallate B, theaflavin digallate, and free theaflavin. Thus, the composition of mouth spray according to the present invention keeps a long duration of masking bad breath and of refreshing effect significantly. Moreover, the composition of mouth spray produces effects such as improvement in a refreshing feeling and pleasant aftertaste, cancellation of throat unpleasantness, and other useful effects that the tea-polyphenol has in nature.

## Description

### BACKGROUND OF THE INVENTION

### (1) Field of the Invention

This invention relates to a composition of mouth spray, more specifically to a composition of mouth spray that has good durability with respect to a masking of bad breath and a refreshing effect and also has a pleasant aftertaste.

### (2) Description of the Related Art

A mouth sprayer is a small pump-type or squeeze-type container having a spray nozzle and contains a liquid (mouth spray) to be sprayed into the mouth. Since the mouth sprayer is convenient to carry, it has been used for various applications for food, non-pharmaceutical and pharmaceutical industries and the like.

In a field of foods or non-pharmaceuticals, refreshing spice such as 1-menthol, mint and peppermint oils containing 1-menthol as a main constituent, 1-carvone, and spearmint oil containing 1-carvone as a main constituent has often been used as an effective ingredient to prepare a composition of mouth spray, which is used to prevent such as bad breath and discomfort. However, these effective ingredients have a disadvantage of poor durability with respect to the masking of bad breath or the refreshing effect.

A tea-polyphenol has been known to have various effects such as odor-eating, antibacterial, anti-influenza and anticarious effect so that the application to pharmaceuticals for mouth such as toothpaste, collutory and mouthwash has hitherto been tried. However, the tea-polyphenol is an essence of sour of tea, giving a strong sour and bitterness when kept solely in the mouth. Consequently, a taste of the tea-polyphenol is not popular and unsuited for a composition of the above pharmaceuticals for mouth.

In addition, being different from toothpaste, collutory and mouthwash, the mouth spray is of an internal use by being sprayed into the mouth so that the suitability to taste is an important factor besides an effect, resulting in that the application of the tea-polyphenol to the composition of pharmaceuticals for mouth has particularly been difficult.

### SUMMARY OF THE INVENTION

It is therefore an object of the present invention to solve the above-mentioned problem and to provide a composition of mouth spray that has good durability with respect to the masking of bad breath and the refreshing effect and also has the pleasant aftertaste.

In order to accomplish the above object, a first aspect of the present invention is to provide a composition of mouth spray comprising tea-polyphenol, 1-menthol and/or 1-carvone.

A second aspect of the present invention is to provide the composition of mouth spray, wherein a concentration of the tea-polyphenol is from 10 to 20000 ppm.

A third aspect of the present invention is to provide the composition of mouth spray, wherein the tea-polyphenol is at least one member selected from the group containing (+) - catechin, (-) - catechin, (+) - gallocatechin, (+) - epigallocatechin, (+) - gallocatechin gallate, (+) - epigallocatechin gallate, (-) - epicatechin, (-) - epicatechin gallate, (-) - catechin gallate, (-) - epigallocatechin, (-) - gallocatechin, (-) - epigallocatechin gallate, (-) - gallocatechin gallate, theaflavin monogallate A, theaflavin monogallate B, theaflavin digallate, and free theaflavin.

The composition of mouth spray according to the present invention keeps a long duration of masking bad breath and of refreshing effect significantly. Moreover, the composition of mouth spray produces effects such as improvement in a refreshing feeling and pleasant aftertaste, cancellation of throat unpleasantness, and other useful effects that the tea-polyphenol has in nature.

### DESCRIPTION OF THE PREFERRED EMBODIMENTS

The composition of mouth spray according to the present invention is not only used to prevent such as bad breath or discomfort from causing, but also applied to a variety of purposes in the field of food, non-pharmaceutical and pharmaceutical industries and the like. An amount of one spraying is normally 10 to 500 microliters.

The 1-menthol used in the present invention may be a synthetic or natural product and may include an isomer thereof. 1-menthol is a main constituent of mint and peppermint oils that may be used as they are in the present invention. The 1-carvone used in the present invention may also be a synthetic or natural product and may include an isomer thereof. A spearmint oil having 1-carvone as a main constituent thereof may also be used.

A concentration of 1-menthol or 1-carvone in the composition of mouth spray according to the present invention is not particularly limited as long as the masking of bad breath and refreshing effect can be obtained, but the concentration of either the single component or the mixture thereof is preferably from 100 to 10000 ppm. When the concentration is lower than 100 ppm, a refreshment due to 1-menthol or 1-carvone is hard to obtain. On the other hand, when the concentration is higher than 10000 ppm, the suitability to taste is inferior caused by too much stimulation and bitterness.

The ingredient of a tea used in the present invention is such as leaf, stem, xylem, root and nut obtained from a camellia sinensis or a mixture thereof, but is usually a tea leaf for drinking. The tea leaf for drinking includes a fermented tea such as black tea and puahru tea, half-fermented tea such as oolong tea and paochoncha, non-fermented tea such as green tea, kamairi (infused in a pot) green tea and houjicha, and a mixture thereof.

The tea-polyphenol used in the present invention may be a tea itself contained tea-polyphenol, an extract obtained by extraction from tea using such as water, hot water, organic solvent and water-containing organic solvent, or a mixture thereof. Such an extract may further be refined to a desirable extent by means of an organic solvent fractionation method or by using an adsorptive resin. These methods have been described in Japanese Patent Publications No. H1-44234, H2-12474 and H2-22755, and in Japanese Patent Applications Laid-Open No. H4-20589, H5-260907 and H8-109178.

The tea-polyphenol used in the present invention may be such as (+) - catechin, (-) - catechin, (+) - gallocatechin, (+) - epigallocatechin, (+) - gallocatechin gallate, (+) - epigallocatechin gallate, (-) - epicatechin, (-) - epicatechin gallate, (-) - catechin gallate, (-) - epigallocatechin, (-) - gallocatechin, (-) - epigallocatechin gallate, (-) - gallocatechin gallate, theaflavin monogallate A, theaflavin monogallate B, theaflavin digallate, and free theaflavin. Selecting from the above group, either the single tea-polyphenol or a mixture of a plurality of the tea-polyphenol may be used. A main constituent of a tea-polyphenol originally from the green tea is (-) - epigallocatechin gallate.

In the present invention, an amount of the tea-polyphenol is defined as an amount of tannin determined by an official method of tannin analysis using iron tartrate.

A concentration of the tea-polyphenol used in the present invention is preferably from 10 to 20000 ppm as the sole tea-polyphenol or the mixture thereof, and more preferably from 50 to 10000 ppm. The desired effect cannot be obtained when the concentration of the tea-polyphenol is lower than 10 ppm, while the suitability to taste becomes inferior caused by giving sour and bitterness when the concentration is higher than 20000ppm.

The composition of mouth spray according to the present invention keeps a long duration of masking bad breath and of refreshing effect significantly. Furthermore, the composition of mouth spray according to the present invention has newly-found effects such as cancellation of throat unpleasantness and pleasant aftertaste, and also has a variety of useful effects that the tea-polyphenol has in nature.

Corresponding to a purpose of use, the composition of mouth spray according to the present invention may contain various additives such as efficacious ingredient, sweetening, solvent, humectant, solubilizer, surface active agent, emulsifier, antioxidant, pH modifier, spice, coloring agent, preservative, buffer, and stabilizer.

For example, the efficacious ingredient may be such as sodium fluoride, sodium monofluorophosphate, tin fluoride, cetylpyridinium chloride, chlorhexyzine gluconate, isopropylmethylphenol, iodine, potassium iodide, povidone-iodine, hinokitiol, tranexamic acid, sodium azulene sulfonate, cuppor chlorophyllin sodium, and vitamin E. The sweetening may be monosaccharide or diasaccharide such as cane sugar, grape sugar, fruit sugar and isomerized sugar, sugar alcohol such as xylitol, erythritol, sorbitol and mannitol, aspartame, stevioside, and saccharin sodium. The humectant may be polyhydric alcohol such as glycerin, polyethylene glycol, and propylene glycol. The solvent may be such as ethanol. The solubilizer may be non-ionic surface-active agent such as polyoxyethylene stiffened castor oil and polyoxyethylene polyoxypropylene glycol. The preservative may be such as benzoic acid, sodium benzoate, paraoxybenzoate ester group, sorbic acid, potassium sorbate, and decomposed pectin. The antioxidant may be such as ascorbic acid, and erysorbic acid. The pH modifier may be such as citric acid, phosphate, malic acid, and sodium hydrogencarbonate. The emulsifier may be such as glycerin fatty acid ester, cane sugar fatty acid ester, sorbitan fatty acid ester, and soybean saponin.

Prescription examples from 1 to 4 of the composition of mouth spray according to the present invention are shown in the following Tables from 1 to 4, respectively. In the tables, polyphenon 60 and polyphenon RB are brand name of products manufactured by Mistui Norin Co., Ltd.

**Table 1**

| | |
|---|---|
| polyphenon 60 (contained 60 wt% or more of green tea-polyphenol, manufactured by Mistui Norin Co., Ltd.) | 0.8 wt% |
| 1-menthol | 0.5 wt% |
| ethanol | 30 wt% |
| polyoxyethylene stiffened castor oil | 2.0 wt% |
| saccharin sodium | 0.1 wt% |
| refined water | remainder |

**Table 2**

| | |
|---|---|
| polyphenon RB (contained 25 wt% or more of black tea-polyphenol, manufactured by Mistui Norin Co., Ltd.) | 0.5 wt% |
| mint oil (contained 35 wt% of 1-menthol) | 0.2 wt% |
| propylene glycol | 20 wt% |
| glycerin | 25 wt% |
| polyoxyethylene stiffened castor oil | 2.0 wt% |
| spice | 1.5 wt% |
| refined water | remainder |

**Table 3**

| | |
|---|---|
| polyphenon 60 (contained 60 wt% or more of green tea-polyphenol, manufactured by Mistui Norin Co., Ltd.) | 0.05 wt% |
| spearmint oil (contained 20 wt% of 1-carvone) | 0.1 wt% |
| xylitol | 20 wt% |
| cane sugar fatty acid ester | 1.5 wt% |
| refined water | remainder |

**Table 4**

| | |
|---|---|
| polyphenon 60 (contained 60 wt% or more of green tea-polyphenol, manufactured by Mistui Norin Co., Ltd.) | 0.01 wt% |
| 1-menthol | 0.3 wt% |
| peppermint oil (contained 30 wt% of 1-menthol) | 0.01 wt% |
| ethanol | 20 wt% |
| sorbitol | 30 wt% |
| polyoxyethylene stiffened castor oil | 2.0 wt% |
| refined water | remainder |

### EXAMPLES

The composition of mouth spray according to the present invention will now be described by the following examples that by no means limit the scope of the invention.

### Comparative Example No. 1

As shown in Table 5, solutions containing 1-menthol of nine kinds of concentrations were prepared and introduced into each mouth spray container having a spray nozzle, amount of one spray of which is 50 µ liters. Then, a sensuous test (A-type) by 10 panelists was conducted. The test (A-type) was performed to evaluate the masking of bad breath and the refreshing effect of the sample solutions by marking scores out of 10 for each panelist, and then scores answered by the 10 panelists were summed up (full marks 100).

The result is shown in Table 6 indicating that the summed score was high when the concentration of 1-menthol was from 100 to 10000 ppm.

**Table 5**

| | |
|---|---|
| 1-menthol | from 0 to 20000 ppm |
| ethanol | 35 wt% |
| polyoxyethylene stiffened castor oil | 3 wt% |
| refined water | remainder |

**Table 6**

| concentration of 1 - menthol (ppm) | masking of bad breath | refreshing effect |
|---|---|---|
| 0 | 0 | 0 |
| 50 | 4 | 11 |
| 100 | 43 | 61 |
| 500 | 66 | 73 |
| 1000 | 80 | 91 |
| 2000 | 87 | 96 |
| 5000 | 90 | 84 |
| 10000 | 89 | 42 |
| 20000 | 25 | 0 |

Polyphenon 100 (brand name of a product manufactured by Mistui Norin Co., Ltd.), active ingredients of which are shown in Table 7, was used as a source of green tea-polyphenol. Mouth spray solutions containing green tea-polyphenol of seven kinds of concentrations were prepared according to a prescription described in Table 8, then a sensuous test (B-type) by 10 panelists was conducted. The test (B-type) was performed to evaluate degrees of sour and bitterness of the sample solutions by marking scores out of 3 for each panelist, and then scores answered by the 10 panelists were summed up (full marks 30). The each score out of 3 corresponds as follows: not felt sour and bitterness 3, felt to some extent 2, felt considerably 1, felt very strongly 0.

Then, a sensuous test (A-type) by 10 panelists was conducted in a similar way as described above.

These results are shown together in Table 9.

**Table 7**

| active ingredient (tea-catechin group) | ratio of components (wt%) |
|---|---|
| (+) - gallocatechin | 1.4 |
| (-) - epigallocatechin | 17.6 |
| (-) - epicatechin | 5.8 |
| (-) - epigallocatechin gallate | 53.9 |
| (-) - epicatechin gallate | 12.5 |
| total | 91.2 |

**Table 8**

| | |
|---|---|
| green tea-polyphenol | from 0 to 20000 ppm |
| ethanol | 35 wt% |
| polyoxyethylene stiffened castor oil | 3 wt% |
| refined water | remainder |

**Table 9**

| concentration of green tea-polyphenol (ppm) | degree of sore and bitterness | masking of bad breath | refreshing effect |
|---|---|---|---|
| 0 | 30 | 0 | 0 |
| 10 | 29 | 0 | 3 |
| 50 | 25 | 13 | 5 |
| 100 | 20 | 20 | 5 |
| 1000 | 9 | 37 | 2 |
| 10000 | 0 | 22 | 0 |
| 20000 | 0 | 0 | 0 |

Polyphenon TF (brand name of a product manufactured by Mistui Norin Co., Ltd.), active ingredients of which are shown in Table 10, was used as a source of black tea-polyphenol. Mouth spray solutions containing black tea-polyphenol of seven kinds of concentrations were prepared according to the same prescription described in Table 8 except that green tea-polyphenol is replaced by black tea-polyphenol, and sensuous tests (A-type and B-type) by 10 panelists were conducted. Both the preparation and the tests were carried out in a similar way to the green tea-polyphenol case as described above. The results are shown in Table 11.

The results shown in tables 9 and 11 reveal as follows: (1) the masking of bad breath that seems to be due to the green or black tea-polyphenol was positively observed, however, (2) the degree of sore and bitterness was significant when the tea-polyphenol concentration was 1000 ppm or higher, and (3) the refreshing effect was hardly observed.

**Table 10**

| active ingredient | ratio of components (wt%) |
|---|---|
| free theaflavin | 6.0 |
| theaflavin monogallate A | 13.4 |
| theaflavin monogallate B | 11.7 |
| theaflavin digallate | 19.5 |
| (+) -catechin | 0.2 |
| (-) - epicatechin | 1.1 |
| (-) - epigallocatechin gallate | 2.8 |
| (-) - epigallocatechin gallate isomer | 0.6 |
| (-) - epicatechin gallate | 2.3 |
| theaflavin isomer | 2.4 |
| total | 60 |

**Table 11**

| concentration of black tea-polyphenol (ppm) | degree of sore and bitterness | masking of bad breath | refreshing effect |
|---|---|---|---|
| 0 | 30 | 0 | 0 |
| 10 | 30 | 0 | 2 |
| 50 | 27 | 11 | 5 |
| 100 | 24 | 19 | 4 |
| 1000 | 11 | 34 | 2 |
| 10000 | 2 | 21 | 1 |
| 20000 | 0 | 0 | 0 |

### Example No. 1

Mouth spray solutions containing 1-menthol of three kinds of concentrations and green tea-polyphenol (polyphenon 100, containing active ingredients shown in Table 7) of six kinds of concentrations were prepared according to a prescription described in Table 12 and a sensuous test (C-type) by 15 panelists was conducted. The test (C-type) includes (1) duration of masking of bad breath and refreshing effect and (2) improvement effect on aftertaste due to refreshed and clean feelings. In the test (1), placebos that include no green tea-polyphenol were prepared besides the sample solutions and a number of panelists who answered that the sample was better than the corresponding placebo with respect to the duration of masking of bad breath and refreshing effect was counted (full marks 15). In the test (2), a number of panelists who answered that the sample had the improvement effect on aftertaste was counted (full marks 15). As for the improvement effect on aftertaste, the sample solution was evaluated to have no such an improvement effect if each panelist felt unpleasant sore or bitterness.

The result is shown in Tables 13 to 15.

**Table 12**

| | |
|---|---|
| 1- menthol | from 500 to 5000 ppm |
| green tea-polyphenol | from 10 to 20000 ppm |
| ethanol | 35 wt% |
| polyoxyethylene stiffened castor oil | 3 wt% |
| refined water | remainder |

**Table 13**

| concentration of 1-menthol (ppm) | concentration of green tea-polyphenol (ppm) | duration of masking of bad breath | duration of refreshing effect | improvement effect on aftertaste |
|---|---|---|---|---|
| 500 | 10 | 9 | 5 | 12 |
| 500 | 50 | 11 | 9 | 14 |
| 500 | 100 | 13 | 12 | 14 |
| 500 | 1000 | 14 | 15 | 13 |
| 500 | 10000 | 14 | 13 | 11 |
| 500 | 20000 | 12 | 2 | 0 |

**Table 14**

| concentration of 1-menthol (ppm) | concentration of green tea-polyphenol (ppm) | duration of masking of bad breath | duration of refreshing effect | improvement effect on aftertaste |
|---|---|---|---|---|
| 2000 | 10 | 8 | 6 | 12 |
| 2000 | 50 | 11 | 8 | 14 |
| 2000 | 100 | 12 | 11 | 14 |
| 2000 | 1000 | 14 | 14 | 15 |
| 2000 | 10000 | 15 | 14 | 11 |
| 2000 | 20000 | 11 | 3 | 0 |

**Table 15**

| concentration of 1-menthol (ppm) | concentration of green tea-polyphenol (ppm) | duration of masking of bad breath | duration of refreshing effect | improvement effect on aftertaste |
|---|---|---|---|---|
| 5000 | 10 | 7 | 4 | 11 |
| 5000 | 50 | 10 | 9 | 13 |
| 5000 | 100 | 12 | 13 | 14 |
| 5000 | 1000 | 14 | 15 | 15 |
| 5000 | 10000 | 15 | 14 | 12 |
| 5000 | 20000 | 13 | 2 | 0 |

The result shown in tables 13 to 15 reveals as follows: (1) by using 1-menthol together with green tea-polyphenol that gives poor masking of bad breath and refreshing effect when used solely, obtained were a significantly long duration of masking of bad breath and refreshing effect as well as a significant improvement effect on aftertaste compared to single usage of 1-menthol, and (2) sour and bitterness due to the green tea-polyphenol were masked by using 1-menthol together with the green tea-polyphenol.

### Example No. 2

Mouth spray solutions containing 1 - menthol (concentration of 2000 ppm) and black tea-polyphenol (polyphenon TF, active ingredients shown in Table 10) of six kinds of concentrations were prepared according to a prescription described in Table 16 and a sensuous test (C-type) by 15 panelists was conducted. The test was carried out in a similar way to the green tea-polyphenol case in Example No. 1 as described above.

The result is shown in Table 17, revealing as follows: (1) by using 1 - menthol together with the black tea-polyphenol, obtained were a significantly long duration of masking of bad breath and refreshing effect as well as a significant improvement effect on aftertaste compared to sole usage of 1-menthol, and (2) sour and bitterness due to the black tea-polyphenol were masked by using 1-menthol together with the black tea-polyphenol.

**Table 16**

| | |
|---|---|
| 1-menthol | 2000 ppm |
| black tea-polyphenol | from 10 to 20000 ppm |
| ethanol | 35 wt% |
| polyoxyethylene stiffened castor oil | 3 wt% |
| refined water | remainder |

**Table 17**

| concentration of 1-menthol (ppm) | concentration of black tea-polyphenol (ppm) | duration of masking of bad breath | duration of refreshing effect | improvement effect on aftertaste |
|---|---|---|---|---|
| 2000 | 10 | 8 | 5 | 11 |
| 2000 | 50 | 10 | 8 | 13 |
| 2000 | 100 | 12 | 11 | 13 |
| 2000 | 1000 | 13 | 13 | 14 |
| 2000 | 10000 | 14 | 11 | 10 |
| 2000 | 20000 | 11 | 2 | 0 |

### Comparative Example No. 2

Mouth spray solutions containinging (-) - epigallocatechin gallate (purity 98 wt% or higher, manufactured by Mistui Norin Co., Ltd.) of seven kinds of concentrations were prepared according to a prescription described in Table 18 and sensuous tests (A-type and B-type) by 10 panelists were conducted in a similar way as comparative example No. 1.

The results are shown together in Table 19.

**Table 18**

| | |
|---|---|
| (-) - epigallocatechin gallate | from 10 to 20000 ppm |
| ethanol | 35 wt% |
| polyoxyethylene stiffened castor oil | 3 wt% |
| refined water | remainder |

**Table 19**

| concentration of ( - ) -epigallocatechin gallate (ppm) | degree of sore and bitterness | masking of bad breath | refreshing effect |
|---|---|---|---|
| 0 | 30 | 0 | 0 |
| 10 | 28 | 0 | 1 |
| 50 | 23 | 16 | 3 |
| 100 | 17 | 23 | 3 |
| 1000 | 6 | 40 | 1 |
| 10000 | 0 | 24 | 0 |
| 20000 | 0 | 0 | 0 |

The results shown in table 19 reveal as follows: (1) the masking of bad breath that seems to be due to (-) - epigallocatechin gallate was positively observed, however, (2) the degree of sore and bitterness was significant when (-) -epigallocatechin gallate concentration was 1000 ppm or higher, and (3) the refreshing effect was hardly observed.

### Example No. 3

Mouth spray solutions containing 1-menthol (concentration 2000 ppm) and ( - ) - epigallocatechin gallate (the same as used in comparative example No. 2) of six kinds of concentrations were prepared according to a prescription described in Table 20 and a sensuous test (C-type) by 15 panelists was conducted. The test was carried out in a similar way to the black tea-polyphenol case in Example No. 2 as described above.

The result is shown in Table 21, revealing as follows: (1) by using 1 - menthol together with (-) -epigallocatechin gallate, obtained are a significantly long duration of masking of bad breath and refreshing effect as well as a significant improvement effect on aftertaste compared to sole usage of 1 - menthol, and (2) sour and bitterness due to (-) - epigallocatechin gallate were masked by using 1-menthol together with (-) - epigallocatechin gallate.

**Table 20**

| | |
|---|---|
| 1-menthol | 2000 ppm |
| (-) - epigallocatechin gallate | from 10 to 20000 ppm |
| ethanol | 35 wt% |
| polyoxyethylene stiffened castor oil | 3 wt% |
| refined water | remainder |

**Table 21**

| concentration of 1-menthol (ppm) | concentration of (-) -epigallo-catechin gallate (ppm) | duration of masking of bad breath | duration of refreshing effect | improvement effect on aftertaste |
|---|---|---|---|---|
| 2000 | 10 | 9 | 7 | 13 |
| 2000 | 50 | 12 | 10 | 14 |
| 2000 | 100 | 13 | 12 | 15 |
| 2000 | 1000 | 15 | 15 | 13 |
| 2000 | 10000 | 14 | 14 | 9 |
| 2000 | 20000 | 10 | 1 | 0 |

## Claims

1. A composition of mouth spray comprising tea-polyphenol, 1-menthol and/or 1-carvone

2. The composition of mouth spray according to claim 1, comprising (a) tea-polyphenol and (b) 1-menthol or 1-carvone.

3. The composition of mouth spray according to claim 1 or claim 2, wherein the concentration of the tea-polyphenol is from 10 to 20000 ppm.

4. The composition of mouth spray according to claim 3, wherein the concentration of the tea-polyphenol is from 50 to 10000 ppm.

5. The composition of mouth spray according to any preceding claim, wherein the total concentration of 1-menthol and/or 1-carvone is from 100 to 10000 ppm.

6. The composition of mouth spray according to any preceding claim, wherein the tea-polyphenol is at least one member selected from the group containing (+)-catechin, (-)-catechin, (+) -gallocatechin, (+) - epigallocatechin, (+)-gallocatechin gallate, (+) - epigallocatechin gallate, (-)-epicatechin, (-)- epicatehin gallate, (-)-catechin gallate, (-) -epigallocatechin, (-)-gallocatechin, (-)-epigallocatechin gallate, (-)-gallocatechin gallate, theaflavin monogallate A, theaflavin monogallate B, theaflavin digallate, and free theaflavin.
